# EUROPEAN PATENT SPECIFICATION

(11) **EP 1 238 097 B1**
(45) Date of publication and mention of the grant of the patent: **23.08.2006**
(21) Application number: 00990692.6
(22) Date of filing: 08.12.2000
(51) Int. Cl.: C12Q 1/26

(54) **DIETHOXYFLUORESCEIN AS INDICATOR FOR CYTOCHROME P450**
DIETHOXYFLUORESCEIN ALS INDIKATOR FÜR CYTOCHROME P450
DIETHOXYFLUORESCEIN EN TANT QU'INDICATEUR POUR CYTOCHROME P450

(30) Priority: 13.12.1999 US 170405 P
(43) Date of publication of application: 11.09.2002
(73) Proprietor: SMITHKLINE BEECHAM PLC, Brentford, Middlesex TW8 9GS (GB); SMITHKLINE BEECHAM CORPORATION, Philadelphia Pennsylvania 19103 (US)
(72) Inventor: BAMBAL, Ramesh, GlaxoSmithKline Pharmaceuticals, King of Prussia, PA 19406 (US); BLOOMER, Jacqueline, Carol, Welwyn, Herfordshire AL6 9AR (GB)
(74) Representative: Thornley, Rachel Mary
(86) International application number: PCT/EP2000/012450
(87) International publication number: WO 2001/044495

(56) References cited:
- WO-A-01/14361
- MILLER A D: "Flow cytometric techniques for measurement of cytochrome P-450 activity in viable cells." METHODS IN CELL BIOLOGY, (1990) 33 71-9. , XP001016404
- WHITE I N H ET AL: "CHARACTERISTICS OF RAT HEPATOCYTES SORTED BY FLUORESCENCE-ACTIVATED FLOW CYTOMETRY EFFECTS OF MIXED FUNCTION OXIDASE INDUCERS" BIOCHEMICAL PHARMACOLOGY, vol. 38, no. 10, 1989, pages 1639-1646, XP001015256 ISSN: 0006-2952
- WHITE I N H ET AL: "FLUORESCENCE-ACTIVATED SORTING OF RAT HEPATOCYTES BASED ON THEIR MIXED FUNCTION OXIDASE ACTIVITIES TOWARDS DIETHOXYFLUORESCEIN" BIOCHEMICAL JOURNAL, vol. 247, no. 1, 1987, pages 23-28, XP001014797 ISSN: 0264-6021

## Description

This invention relates to the use of a fluorescein derivative as a substrate for cytochrome P450 enzymes.

The majority of metabolism based drug interactions are a result of inhibition of cytochrome P450 enzymes. Drug interactions involving individual P450 enzymes can be predicted using *in vitro* methods. Typical *in vitro* P450 enzyme assays involve incubation of an appropriate substrate with a source of enzyme. Traditionally, time consuming chromatographic methods have been used for metabolite detection in these incubations. More recently the availability of fluorimetric plate readers has facilitated the higher throughput of enzyme assays in general. Adapting P450 assays to fluorescent plate reader technology requires the identification of substrates with appropriate fluorescent products for individual enzymes. Among the xenobiotic-metabolising cytochromes P450, CYP3A4 is one of those commonly responsible for the metabolism of drugs. CYP2C8 has also been identified as being responsible for the metabolism of some drugs.

Resorufin benzyl ether has been described for high throughput CYP3A4 inhibition screening (Crespi *et al, Anal. Biochem*., 1997, **248,** 188-190). However, the rate of resorufin benzyl ether metabolism by CYP3A4 is low, therefore a more appropriate CYP3A4 substrate is required to enable higher throughput inhibition screening. The use of dibenzylfluorescein as a substrate for CYP3A4 and CYP2C8 has also been disclosed, Crespi *et al*, "Innovative Technologies for the Study of Drug Metabolism", presented at the 7th European ISSX Meeting, Budapest, Hungary, August 22-26, 1999. However this assay has a low signal to noise ratio, therefore, a more appropriate CYP3A4 or CYP2C8 substrate is required to improve the sensitivity for inhibition screening.

Miller, *Anal. Biochem*., 1983, **133**, 46-57 describes the compound diethoxyfluorescein and the investigation of its metabolism by mouse liver homogenates and cultured mouse hepatoma cells. Miller concludes that diethoxyfluorescein is not useful for the detection of inducible mouse cytochromes P450.

WO 00/44933 discloses a resorufin derivative as a CYP3A4 substrate.

Diethoxyfluorescein has now been identified as a general substrate for human cytochrome P450 enzymes. In particular diethoxyfluorescein is an improved substrate for human CYP3A4 and CYP2C8, which is of use for configuring high throughput inhibition screening assays.

According to the invention there is provided an assay for identifying inhibitors of a human cytochrome CYP3A4 or CYP2C8P450 enzyme which comprises contacting the enzyme and a compound of formula (I): 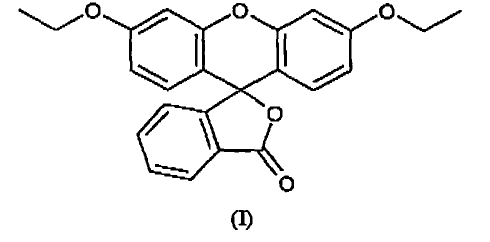 with a test compound and measuring inhibition of O-dealkylation of the compound of formula (I) by the enzyme.

The human cytochrome P450 enzyme used in the process of the invention is preferably CYP3A4 or CYP2C8, more preferably CYP3A4.

Generally the rate of O-dealkylation of the compound of formula (I) in the absence of test compound will be known, as will the extent of O-dealkylation at given time points. The assay may identify inhibition of O-dealkylation continuously or at specified time points.

O-Dealkylation of the compound of formula (I) following incubation with e.g. CYP3A4 or CYP2C8 gives an O-dealkylated compound which exists in tautomeric forms as shown below:

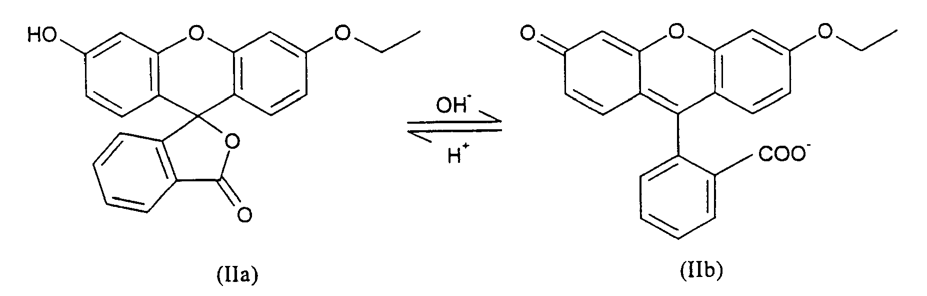

The nonfluorescent lactone (IIa) predominates at low pH whilst the fluorescent charged form (IIb) predominates at neutral to basic pH.

The readily quantifiable fluorescent product of formula (IIb) can be scanned with suitable excitation and emission wavelengths, for example an excitation wavelength of 485 nm and an emission wavelength of 530 nm. Inhibition of O-dealkylation of the compound of formula (I) by the enzyme is preferably measured by quantifying the compound of formula (IIb).

The assay may be carried out either in solution or utilising a solid support in which case the enzyme may be attached to a solid support. When the assay is carried out in solution suitable solvents include methanol and acetonitrile.

The assay is preferably performed in a solution buffered to a pH of 7.4 or 7.5, e.g. using a potassium phosphate or Tris HCl buffer. The assay may also be performed in potassium phosphate buffer containing 10 mM MgCl₂. The assay is preferably performed at a temperature of 37°C.

The test compound may be pre-incubated with enzyme prior to the addition of the substrate, or alternatively the substrate may be added simultaneously with the test compound. Final concentrations of enzyme and substrate are calculated so as to achieve a suitable rate of processing for carrying out the assay. If desired, the reaction may be stopped, for example by addition of acid or solvent.

As will be apparent to those skilled in the art cofactors for the human cytochrome P450 enzyme will be present in the assay system, cofactors for human cytochrome P450 enzymes are NADP, glucose-6-phosphate and glucose-6-dehydrogenase. NADH or NADPH may be used instead of NADP. The assay may conveniently be initiated by addition of the cofactor solution, preferably prewarmed to 37°C, to the test compound / enzyme / substrate mixture.

The fluorescent product of formula (II) may be analysed using any conventional system of fluorescence detection, for example a multi-well plate/fluorescent plate reader.

The compound of formula (I) may be prepared according to the procedure described by Miller, *Anal. Biochem*., 1983, **133**, 46-57.

Since the inhibition of cytochrome P450 enzymes is often the mechanism for drug/drug interactions, the assay according to the invention is particularly useful for identifying compounds which may give rise to adverse drug/drug interactions. The assay can therefore be used in combination with the chemical modification of test compounds to increase a test compounds potential for use as a pharmaceutical.

Thus the assay of the present invention may be of benfit in efforts to reduce the human cytochrome P450 enzyme inhibitory activity of a compound identified as an inhibitor of a human cytochrome CYP3A4 or CYP2C8 P450 enzyme in the assay described above, by producing a chemically modified version of the test compound in which the functionality suspected to be responsible for the human cytochrome P450 enzyme inhibition is eliminated or changed.

The chemical modification of test compounds can be performed using techniques well known to those skilled in the art.

The invention is illustrated by the following example and figures.
Figure 1 shows the ketoconazole inhibition of diethoxyfluorescein metabolism by CYP3A4.
Figure 2 shows the quercitin inhibition of diethoxyfluorescein metabolism by CYP2C8.

### Example 1

### Assay methodology for CYP3A4

### Materials:

500 µM diethoxyfluorescein, (i.e. 0.194 mg/mL in acetonitrile) - store at approx. -20°C in the dark
2 % (w/v) NaHCO₃ - store at approx. 4°C
50 mM potassium phosphate buffer, pH 7.4
Freshly prepared cofactor solution:- approx. the following per mL of 2 % (w/v) NaHCO₃
1.7 mg NADP, monosodium salt
7.8 mg glucose-6-phosphate, monosodium salt
6 Units glucose-6-phosphate dehydrogenase, Type VII from Bakers Yeast

### Method:

1) Pre-warm the plate reader oven to 37°C and pre-warm the lamp for at least 10 minutes.
2) Mix 2.5 µL of 500 □M diethoxyfluorescein, 4 µL (40 µg) CYP3A4 microsomal protein and 213.5 µL buffer per incubate (giving 5 µM diethoxyfluorescein and 160 µg/mL protein final concentration).
3) To each well of a 96-well plate add 220 µL of incubation mix and 5 µL of test compound solution (or 5 µL of appropriate solvent for control wells - methanol or acetonitrile may be used).
4) Pre-incubate the multi-well plate in the plate reader at 37°C for 5 minutes. Pre-warm the cofactor solution at 37°C for 5 minutes.
5) Add 25 µL cofactor solution to each well and scan with an excitation wavelength of 485 nm and an emission wavelength of 530 nm with a gain of 80. Scan for 10 cycles at 1 minute intervals.

### Results

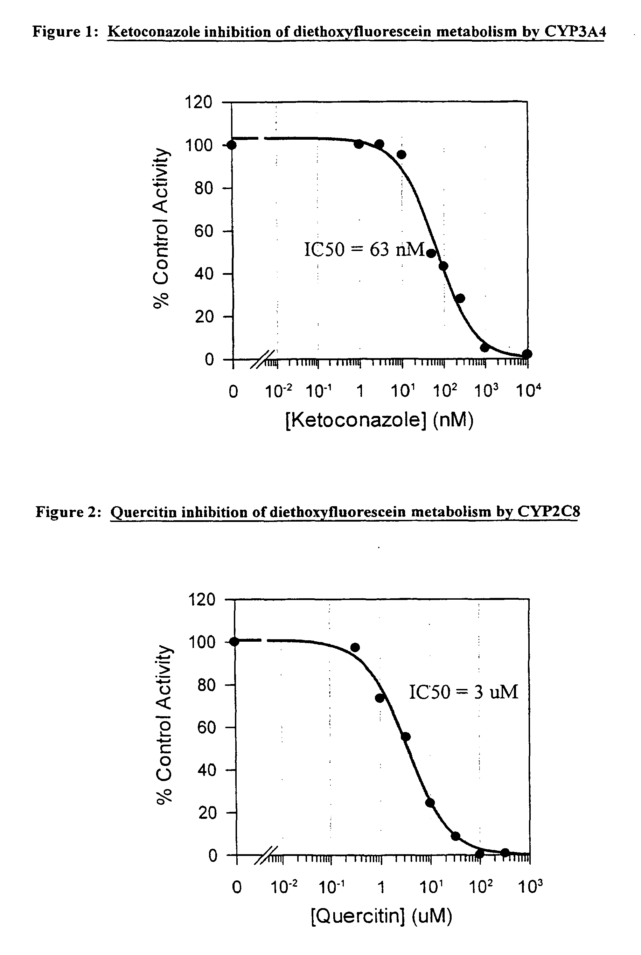

Confirmation of diethoxyfluorescein as a CYP3A4 substrate was achieved using ketoconazole, a diagnostic CYP3A4 inhibitor (Baldwin *et al, Xenobiotica*, 1995, **25,** 261-270). With ketoconazole, diethoxyfluorescein was inhibited with an IC₅₀ of 63 nM (Figure 1), an inhibition value typical of other, well characterised, CYP3A4 substrates.

### Example 2

### Assay methodology for CYP2C8

### Materials:

400 µM diethoxyfluorescein, (i.e. 0.155 mg/mL in acetonitrile) - store at approx. -20°C in the dark
2 % (w/v) NaHCO₃ - store at approx. 4°C
50 mM potassium phosphate buffer, pH 7.4
Freshly prepared cofactor solution:- approx. the following per mL of 2 % (w/v) NaHCO₃
1.7 mg NADP, monosodium salt
7.8 mg glucose-6-phosphate, monosodium salt
6 Units glucose-6-phosphate dehydrogenase, Type VII from Bakers Yeast

### Method:

1) Pre-warm the plate reader oven to 37°C and pre-warm the lamp for at least 10 minutes.
2) Mix 2.5 µL of 400 □M diethoxyfluorescein, 5 µL (50 µg) CYP2C8 microsomal protein and 212.5 µL buffer per incubate (giving 4 □M diethoxyfluorescein and 200 µg/mL protein final concentration).
3) To each well of a 96-well plate add 220 □L of incubation mix and 5 µL of test compound solution (or 5 µL of appropriate solvent for control wells - methanol, DMSO or acetonitrile may be used).
4) Pre-incubate the multi-well plate in the plate reader at 37°C for 5 minutes. Pre-warm the cofactor solution at 37°C for 5 minutes.
5) Add 25 µL cofactor solution to each well and scan with an excitation wavelength of 485 nm and an emission wavelength of 530 nm with a gain of 80. Scan for 10 cycles at 1 minute intervals.

### Results

Confirmation of diethoxyfluorescein as a CYP2C8 substrate was achieved using quercitin, a CYP2C8 inhibitor (Rahman *et al*, *Cancer Research,* 1994, **54**(1), 5543-5546). With quercitin, diethoxyfluorescein metabolism was inhibited with an IC₅₀ of 3 µM (Figure 2), an inhibition value typical of other, well characterised, CYP2C8 substrates.

## Claims

1. An assay for identifying inhibitors of a human cytochrome CYP2C8 or CYP3A4 P450 enzyme which comprises contacting the enzyme and a compound of formula (I): 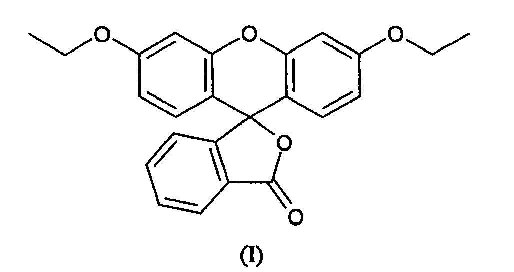 with a test compound and measuring inhibition of O-dealkylation of the compound of formula (I) by the enzyme.

2. The assay according to claim 1 wherein the human cytochrome P450 enzyme is CYP3A4.

3. The assay according to any one of the preceeding claims wherein inhibition of O-dealkylation of the compound of formula (I) by the enzyme is measured by quantifying the compound of formula (IIb):

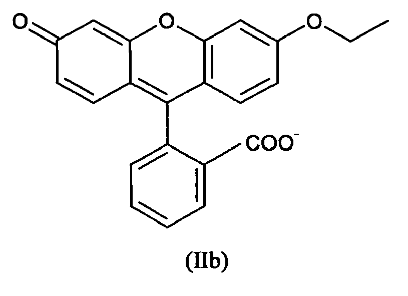

4. The assay according to claim 3 wherein the compound of formula (IIb) is quantified by fluorescence detection.

5. The assay according to claim 4 wherein the compound of formula (IIb) is quantified by scanning at excitation wavelength of 485 nm and an emission wavelength of 530 nm.

## Patentansprüche

1. Test zur Identifizierung von Inhibitoren eines menschlichen Cytochrom CYP2C8 oder CYP3A4 P450-Enzyms, der das Inkontaktbringen des Enzyms und einer Verbindung der Formel (I): 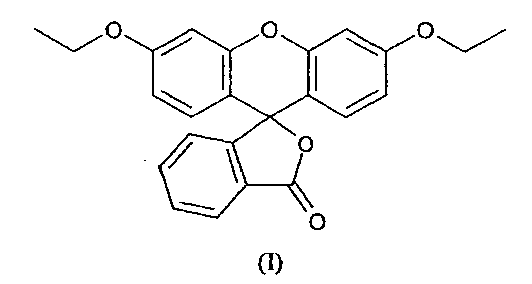 mit einer Testverbindung und das Messen der Hemmung der O-Dealkylierung der Verbindung der Formel (I) durch das Enzym umfasst.

2. Test gemäß Anspruch 1, wobei das menschliche Cytochrom P450-Enzym CYP3A4 ist.

3. Test gemäß einem der vorstehenden Ansprüche, wobei die Hemmung der O-Dealkylierung der Verbindung der Formel (I) durch das Enzym durch quantitative Bestimmung der Verbindung der Formel (IIb) gemessen wird:

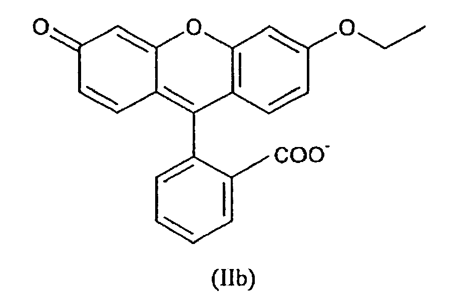

4. Test gemäß Anspruch 3, wobei die Verbindung der Formel (IIb) quantitativ durch Fluoreszenzmessung bestimmt wird.

5. Test gemäß Anspruch 4, wobei die Verbindung der Formel (IIb) durch Scannen bei einer Anregungswellenlänge von 485 nm und einer Emissionswellenlänge von 530 nm quantitativ bestimmt wird.

## Revendications

1. Analyse pour identifier des inhibiteurs d'une enzyme cytochrome humaine CYP2C8 ou CYP3A4 P450, qui comprend la mise en contact de l'enzyme et d'un composé de formule (I) : 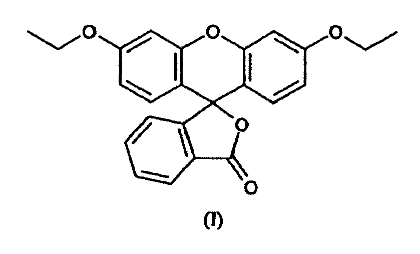 avec un composé d'essai et la mesure de l'inhibition de la O-désalkylation du composé de formule (I) par l'enzyme.

2. Analyse suivant la revendication 1, dans laquelle l'enzyme cytochrome P450 humaine est la CYP3A4.

3. Analyse suivant l'une quelconque des revendications précédentes, dans laquelle l'inhibition de la O-désalkylation du composé de formule (I) par l'enzyme est mesurée en quantifiant le composé de formule (IIb) :

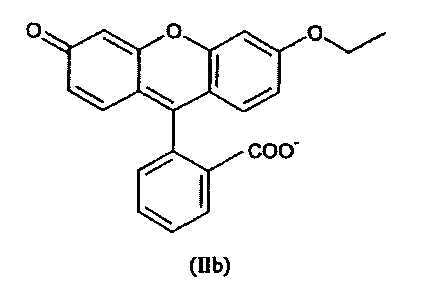

4. Analyse suivant la revendication 3, dans laquelle le composé de formule (IIb) est quantifié par détection de fluorescence.

5. Analyse suivant la revendication 4, dans laquelle le composé de formule (IIb) est quantifié par balayage à une longueur d'onde d'excitation de 485 nm et une longueur d'onde d'émission de 530 nm.
